# EUROPEAN PATENT APPLICATION

(11) **EP 0 948 945 A2**
(43) Date of publication of application: **13.10.1999**
(21) Application number: 99104005.6
(22) Date of filing: 12.03.1999
(51) Int. Cl.: A61F 2/06

(54) **Endovascular prothesis with fixation means**

(30) Priority: 13.03.1998 AR 9801144
(71) Applicant: Parodi, Juan Carlos, Federal Capital (AR)
(72) Inventor: Parodi, Juan Carlos, Federal Capital (AR)
(74) Representative: HOFFMANN - EITLE

(57) **Abstract**

An endovascular prosthesis with suture holder comprises prosthetic body (b) on the internal surfaces (2) of which a number of connectors (4) are distributed and attached that, on the one hand, connect respective semi-flexible cables (b) ending in external rotary control ends (9'), while, on the other hand, they are passed through by respective spiral sutures (6) controlled by said semi-flexible cables (b). Once prosthetic body (b) is expanded, its external surfaces (3) are in contact with vascular walls (1). With this arrangement, the rotary actuation of semi-flexible cables (c) permits penetration ends (6') of spirals (6) to pass through vascular walls (5). This penetration continues until anchoring ends (7) of above-cited spirals (6) stop against bases (4') of respective connectors (4). In this manner, a firm union is created between vascular channel (a) and prosthetic body (b).

## Description

This invention consists of an endovascular device with suture holder with the features of the preamble of claim 1. Such a device permits the creation of a firm union between the prosthesis and the vascular walls with no need to introduce suture applicator devices into the vascular channel.

To treated diseases of the arterial and venous channels, recourse is had to endovascular treatments based on the application of expandable prostheses or endoluminal expanders that permit the affected vascular walls to be covered and thus provide an effective solution to problems that would otherwise cause a high rate of mortality.

Nonetheless, the new application techniques and devices must solve problems such as how to avoid vascular dilation subsequent to the treatment causing periprosthetic losses resulting in consequences difficult to solve.

To avoid this type of problem, internal sutures are used that prove highly effective in obtaining a firm union between the vascular walls and the body of the prosthesis.

These endovascular sutures consist of metal spirals provided with an sharp penetrating end and an anchoring end that are applied by means of devices equipped with rotary applicator heads. In this manner, the above-mentioned spirals pass through the walls of the prosthesis first, and then the vascular walls, achieving a very firm union by means of which the sutured prosthesis conveniently accompanies the dilation of the vascular channel.

However, the known devices used for this type of treatment include a set of means such as distal or proximal inflatable balloons, tubular parts that provide a surgical method in which a rotary pusher for the above-cited spiral sutures, auxiliary lines, etc., can function.

In addition, these devices have a function based on the fact that the operations for introducing the sutures are performed inside the vascular channel.

This device permits disposing of devices such as those mentioned, since both the sutures and the means of application are mounted on the prosthesis itself.

Basically it comprises a prosthetic body over the internal surfaces of which a number of connectors are distributed and attached; on the one hand, they connect the respective semi-flexible cables to external, rotary control ends while, on the other hand, they are passed through by respective spiral sutures controlled by said semi-flexible cables.

That is, the spiral sutures are already applied to the prosthetic body, as are the semi-flexible applicator cables. Once the prosthetic body is expanded, its external surfaces are backed against the vascular walls. With this arrangement, the rotary actuation of the semi-flexible cables permit the penetrating ends of the spirals to pass through the vascular walls.

This penetration continues until the anchoring tips of the above-cited spirals meets the bases of their respective connectors. In this manner, a firm union is created between the vascular channel and the prosthetic body and the semi-flexible cables are simply disconnected and withdrawn.

This creates an endovascular prosthesis that does not require the introduction of additional applicators, that simplifies placement operations and is more economical than other devices currently in use.

For greater clarity and better comprehension of the subject of the invention, it is illustrated with various figures showing one of the preferred methods of embodiment, all as a simple illustrative and not limitative example.

Figure 1 is a perspective view of the interior passage of the prosthetic body showing the semi-flexible cables and corresponding connectors.

Figure 2a is a perspective view of a connector with its respective semi-flexible cable. A spiral suture appears through the mouth of the connector.

Figure 2b is a perspective view, as in Figure 2a, but showing how the rotation of the cable causes the spiral to advance.

Figure 3 is a perspective view of the internal surface of the prosthetic body.

Figure 4 is a perspective view of the external surface of the prosthetic body.

Figure 4a is a longitudinal cross-section of the prosthetic body and the vascular channel for the application once the spiral sutures have been applied and the respective semi-flexible cables disconnected.

Figure 5a is a perspective detail of a connector and the applicator end of the semi-flexible cable in which the application movements of the spiral suture are shown.

Figure 5b is a detail, as in Figure 5a, but showing the disconnection movements of the applicator end of the semi-flexible cable.

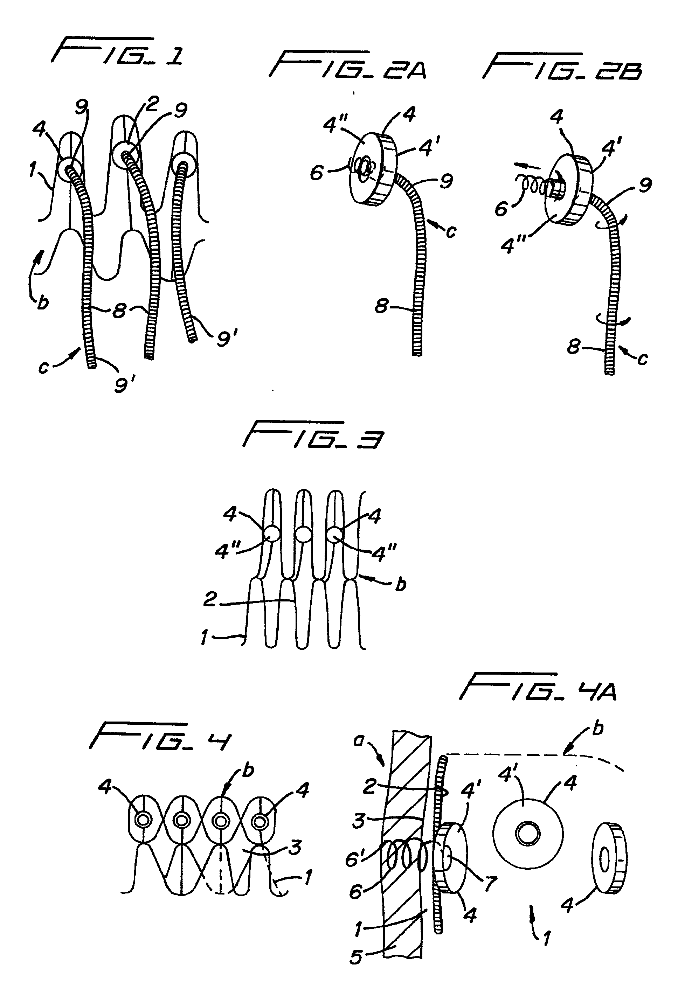

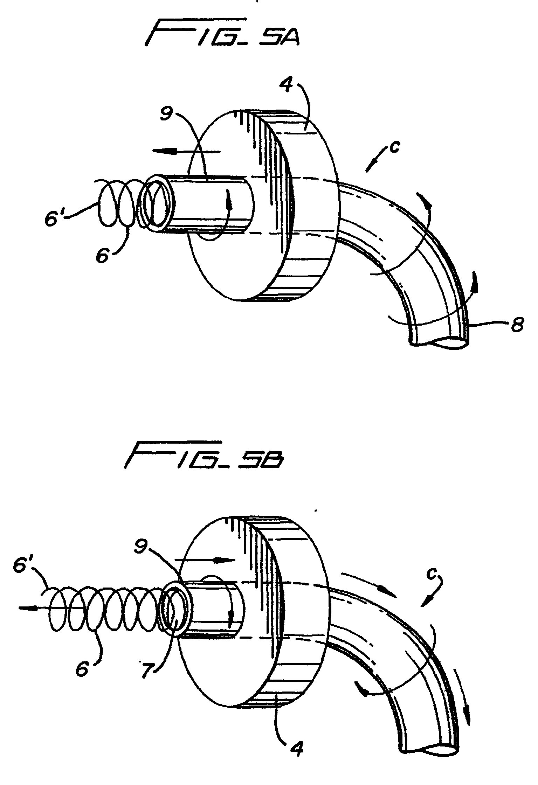

In the various Figures, the same reference numbers indicate the same or similar parts, and the sets of various components are indicated by letters.

### List of the Main References:

(a) vascular channel for application
(b) prosthetic body
(c) semi-flexible cables
(1) cylindrical walls of prosthetic body (b)
(1') prosthetic passage delimited by cylindrical walls (1)
(2) internal surfaces of cylindrical walls (1)
(3) external surfaces of cylindrical walls (1)
(4) connector in form of small disk
(4') base of connector (4)
(4") mouth of connector (4)
(5) vascular walls of vascular channel (a)
(6) spiral sutures
(6') sharpened penetrating end of spiral suture (6)
(7) anchoring end of spiral suture (6)
(8) long body of semi-flexible cable (b)
(9) rotary applicator end, disconnectable from cable (b)
(9') external rotary control end of cable (b).
For the purposes specific, this endovascular prosthesis with suture holder is of the type that, since it can be introduced endolumininally into vascular channel (a) for the application, comprises prosthetic body (b) with cylindrical walls (1) and open bases, the is expandable radially up to the limit at which its external surfaces (3) come into contact with damaged vascular walls (5) of the above-cited vascular channel (a), with internal surfaces (2) of said body (b) forming prosthetic circulatory passage (1'); said endovascular prosthesis is characterized in that it comprises:
a) a number of connectors (4) that, distributed such that they encircle the cylindrical walls (1) of prosthetic body (b), are attached to internal surfaces (2) of said cylindrical walls (1);
b) in each connector (4), rotary end (9), which is disconnectable from respective semi-flexible cable (c) for applying sutures (6), which cable (c) extends beyond said prosthetic passage (1'), ending in an external rotary control end (9'); and
c) passing through each connector (4) and cylindrical wall (1) adjacent to said prosthetic body (b), respective spiral suture (6), the anchoring ends (7) of which are inserted in said rotary end (9) of its respective semi-flexible cable (b).

This invention consists of an endovascular prosthesis with suture holder that, in general terms, comprises prosthetic body (b) on internal surfaces (2) of which a number of connectors (4) are distributed and attached that, on the one hand, connect respective semi-flexible cables (b) ending in external rotary control ends (9'), while, on the other hand, they are passed through by respective spiral sutures (6) controlled by said semi-flexible cables (b).

More particularly, the endovascular prosthesis consists of prosthetic body (b) with cylindrical walls (1) and open bases that can be introduced into vascular channel (a) for the application until it reaches its position with regard to the damaged vascular walls (5). It is a prosthetic body (5) that can be expanded radially up to the limit at which its cylindrical walls (1) contact above-cited vascular walls (5).

In various methods of embodiment, prosthetic body (b) can be composed of a thermo-expandable material or a material expandable by means on an inflatable balloon or similar device.

Cylindrical walls (1) of prosthetic body (b) are delimited by external (3) and internal (2) surfaces. It is precisely on the internal walls (2) that a number of connectors (4) are attached that, in the method of embodiment described, consist of parts in the shape of small disks, the mouth (4") of which is placed against internal surface (2), while its base (4') remains exposed inside prosthetic passage (1') made up of cylindrical walls (1). These connectors (4) are distributed inside cylindrical walls (1), lining them in a circular manner.

At each connector (4), there terminates respective semi-flexible cable (c) for application of sutures (6), for which reason, for each connector (4) available, the prosthesis has a semi-flexible cable (c). This latter (c) consists of long body (8) with applicator end (9) that can be disconnected from connector (4) to which it is connected. Starting from this disconnectable applicator end (9), semi-flexible cable (c) extends beyond the prosthetic passage (1') until it projects from vascular channel (a), ending in an external control end (9') which can be connected to a rotary device.

Moreover, each connector (4) is passed through by respective spiral suture (6) provided with sharpened penetrating end (6') and, at the opposite end, with anchoring end (7). These spiral sutures (6) pass through connectors (4) and prosthetic walls (1) equipped at two alternative end positions: one for prosthetic positioning, at which anchoring ends (7) are inserted into applicator ends (9) of their respective semi-flexible cables (c); and the other position for placement of the prosthesis, in which sharpened ends (6') of said spirals (6) protrude through prosthetic walls (1) and pass through vascular walls (5) for the application.

In this second, prosthetic-placement position, bases (4') of connectors (4) constitute a penetration stop for anchoring ends (7) of spiral sutures (6).

### The unit functions in the following manner:

By means of any suitable introductory device, the endovascular prosthesis is introduced through vascular channel (a) for the application until it reaches its position with regard to damaged vascular walls (5).

Under these conditions, prosthetic body (b) is contracted and spiral sutures (6) passed through connectors (4) and cylindrical walls (1) of prosthetic body (b), although without projecting externally therefrom further than its external surface (3).

Once prosthetic body (b) is expanded, its external surfaces (3) are in contact with vascular walls (1). With this arrangement, the rotary actuation of semi-flexible cables (c) permits penetration ends (6') of spirals (6) to pass through vascular walls (5). This penetration continues until anchoring ends (7) of above-cited spirals (6) stop against bases (4') of respective connectors (4). In this manner, a firm union is created between vascular channel (a) and prosthetic body (b).

It is indubitable that, once this device is used in practice, changes can be made in certain design and form details without escaping from the fundamental principles substantiated clearly in the following Claims.

## Claims

1. ENDOVASCULAR PROSTHESIS WITH SUTURE HOLDER: of the type that, since it can be introduced endoluminally into a vascular channel for application, comprises a prosthetic body with cylindrical walls and open bases that can be radially expanded up to the limit at which its external surfaces contact the damaged vascular walls of the above-cited vascular channel, with the internal surfaces of said body forming a prosthetic circulatory passage; **characterized in that** it comprises:
a) a number of connectors that, distributed such that they encircle the cylindrical walls of the prosthetic body, are attached to the internal surfaces of said cylindrical walls;
b) in each connector, the rotary end disconnectable from a respective semi-flexible cable as an applicator of sutures, which cable extends beyond said prosthetic passage, terminating in an external rotary control end; and
c) passing through each connector and the adjacent cylindrical wall of said prosthetic body, a respective spiral suture, the anchoring end of which is inserted in said rotary end of its respective semi-flexible cable.

2. ENDOVASCULAR PROSTHESIS WITH SUTURE HOLDER, pursuant to Claim 1: **characterized in that** the connectors consist of parts in the shape of small disks, the base of which constitutes a penetration stop for the anchoring end of the respective spiral suture.

3. ENDOVASCULAR PROSTHESIS WITH SUTURE HOLDER, pursuant to Claim 1: **characterized in that** the external control ends of the semi-flexible cables form connecting ends with a rotary device.

4. ENDOVASCULAR PROSTHESIS WITH SUTURE HOLDER, pursuant to Claim 1: **characterized in that** the spiral sutures pass through the connectors and the walls of the prosthetic body between two alternative end positions: one for prosthetic positioning, in which its anchoring ends are inserted in the ends of its respective semi-flexible cables; and another prosthetic-placement position in which the sharp ends of said spirals protrude through the prosthetic walls and pass through vascular walls for the application.

5. ENDOVASCULAR PROSTHESIS WITH SUTURE HOLDER, pursuant to Claim 1: **charactcrized in that** the walls of the prosthetic body are composed of a thermo-expandable material.

6. ENDOVASCULAR PROSTHESIS WITH SUTURE HOLDER, pursuant to Claim 1: **characterized in that** the connectors encircle the interior of the cylindrical walls, lining them in circular fashion.
